**Europäisches Patentamt**

**European Patent Office**

**Office européen des brevets**

(11) Veröffentlichungsnummer: **0 293 506**

**A1**

## EUROPÄISCHE PATENTANMELDUNG

(21) Anmeldenummer: 87108059.4

(22) Anmeldetag: 04.06.87

(51) Int. Cl.⁴: **A61B 5/02**

(43) Veröffentlichungstag der Anmeldung:
**07.12.88 Patentblatt 88/49**

(84) Benannte Vertragsstaaten:
**AT BE CH DE ES FR GB GR IT LI LU NL SE**

(71) Anmelder: **pfm Plastik für die Medizin GmbH**
**Unterbuschweg 45**
**D-5000 Köln 50 (Sürth)(DE)**

(72) Erfinder: **Rieck, Ralf**
**Am Thieleshof 52**
**D-4006 Erkrath 1(DE)**

(74) Vertreter: **Hanewinkel, Lorenz, Dipl.-Phys.**
**Patentanwalt Ferrariweg 17a**
**D-4790 Paderborn(DE)**

(54) **Ventilschleuse für medizinische Zwecke.**

(57) Die Ventilschleuse ist insbesondere für die Herzzeitvolumenmessung oder Flüssigkeitsspülung bestimmt und hat ein eine Saug- und Druckkammer (2) aufweisendes Gehäuse (1), an dem ein Anschluß (3) für einen Katheter (4), ein Anschluß (5) für eine Flüssigkeits-Zuführleitung (6), ein Anschluß (7) für eine Spritze (8) und ein Anschluß (9) für ein Temperatur-Meßorgan (10,15) oder eine Infusionsleitung oder ein Zuspritzteil oder eine Verschlußkappe (19) angeordnet sind. Dem Anschluß (5) für die Flüssigkeits-Zuführleitung (6) und dem Anschluß (3) für den Katheter (4) ist innerhalb der Saug- und Druckkammer (2) jeweils ein Rückschlagventil (11,12) zugeordnet

Mit dieser Ventilschleuse ist unter Vermeidung von Transport-und Zeitverlusten und Handhabungsunterschieden eine sehr genaue Temperaturmessung möglich.

Die Schleuse stellt ein in sich geschlossenes System dar und schließt somit Infektionsgefahren weitgehendst aus.

Fig.1

## Ventilschleuse für medizinische Zwecke

Die Erfindung bezieht sich auf eine Ventilschleuse für medizinische Zwecke, insbesondere für die Herzzeitvolumenmessung oder Flüssigkeitsspülung.

Die Kälteverdünnungsmethode dient schon seit vielen Jahren zur Bestimmung der Blutstromstärke und die Messungen können ohne Belastung für den Patienten in kurzen Abständen wiederholt werden.

Seit einigen Jahren stehen elektronische Meßgeräte zur Verfügung, die mittels Analogrechner eine sofortige Auswertung der Verdünnungskurve ermöglichen und das Ergebnis digital angeben.

Als Indikatorsubstanz wird über einen Venenkatheter in oder vor dem rechten Vorhof ein bestimmtes Volumen gekühlter Flüssigkeit injiziert. Die dadurch bewirkte Temperaturveränderung des Blutes wird auf der arteriellen Seite gemessen. Zu diesem Zweck schob man bisher über eine A. femoralis oder eine A. cubitales eine Thermistorsonde bis in den Aortenbogen vor. Die neuen Katheterisierungsmethoden ohne Röntenkontrolle (Mikro- und Ballon-Einschwemmkatheter) ermöglichen jedoch eine venöse Einführung von ein- oder mehrlumigen Thermistorkathetern bis in die Pulmonalarterie. Die HZV-Messung ist durch diese Erleichterung zu einer Routine-Untersuchung geworden, die am Patientenbett vorgenommen werden kann.

An dem Thermistor wird ein Computer angeschlossen, der nach Eingabe des Injektionsvolumens und der Temperatur-Differenz zwischen der Injektionslösung und dem Blut des Patienten sofort die Temperatur-Zeit-Kurve auswertet und den Wert des Herzzeitvolumens in Liter/Minute anzeigt.

Die Indikatorzugabe muß möglichst schnell (unter 3 s) erfolgen, um Temperaturverluste im Katheter zu vermeiden.

Um die Herzzeitvolumenmessung noch genauer gestalten zu können und vergleichbare Werte von Klinik bzw. von der die Messung durchführenden Person zu Person zu erhalten, und das gesamte Meßsystem zu standardisieren, liegt der Erfindung die Aufgabe zugrunde, eine einfach aufgebaute und leicht zu handhabende Ventilschleuse zu schaffen, die ein geschlossenes Meßsystem bildet und gleichzeitig für Spülvorgänge einsetzbar ist und die Infektionsgefahr auf ein Minimum reduziert.

Diese Aufgabe wird erfindungsgemäß durch die kennzeichnenden Merkmale des Patentanspruches 1 gelöst, wobei noch die in den Unteransprüchen aufgeführten Gestaltungsmerkmale vorteilhafte Weiterbildungen der Aufgabenlösung darstellen.

Der Gegenstand der Erfindung erstreckt sich nicht nur auf die Merkmale der einzelnen Ansprüche, sondern auch auf deren Kombination.

Durch die erfindungsgemäße Ventilschleuse werden folgende Vorteile erzielt:

1. An die Ventilschleuse wird für die Herzzeitvolumenmessung ein Venenkatheter/Einschwemmkatheter, eine Flüssigkeitszuführleitung und eine Spritze angeschlossen und der in der Ventilschleuse vorgesehene Temperaturfühler mit seiner elektrischen Leitung an einen Computer angeschlossen, so daß die Ventilschleuse mit den vorgenannten Einrichtungen ein geschlossenes Meßsystem bildet, das für die Dauer der in gewissen Abständen erfolgenden Messungen in sich geschlossen bleibt, wodurch eine Infektionsgefahr auf ein Minimum reduziert wird.

2. Durch die Spritze wird die Indikatorflüssigkeit aus dem gekühlten Behälter über die Zuführleitung in die Ventilschleuse hineingesaugt und dort direkt in ihrer Temperatur durch den Temperaturfühler gemessen, der die Temperatur an den Computer weitergibt, so daß genau festgestellt werden kann, welche Temperatur die zu injizierende Flüssigkeit hat - dadurch werden Temperaturverluste durch Transportwege, Zeit und unterschiedliche Handhabung durch das Personal weitestgehend ausgeschlossen und die Temperatur der Flüssigkeit wird in der Ventilschleuse wesentlich genauer gemessen.

3. Da dem Bedienungspersonal vorgegeben ist, in welcher Zeit die Flüssigkeit aus der Ventilschleuse heraus über den Katheter durch die Spritze in die Herzkammer gedrückt werden soll, werden auch zeitlich gleiche Behandlungsphasen erreicht die wesentlich zur genaueren Messung beitragen. Hierdurch ist eine Standardisierung des Vorganges der Herzzeitvolumenmessung erreicht worden und es werden Fehlerquellen ausgeschlossen; auch werden hierdurch vergleichbare Werte von Klinik zu Klinik und von Personal zu Personal erreicht.

4. Neben der Herzzeitvolumenmessung ermöglicht die Ventilschleuse auch die Durchführung von Spülvorgängen, indem dann die Ventilschleuse ohne Temperaturfühler ausgestattet und der Anschluß für den Temeraturfühler durch einen Stopfen verschlossen ist. An diesen, für den Temperaturfühler vorgesehenen Anschluß läßt sich aber auch eine Infusionsleitung anschließen oder der Stopfen kann als Durchstechteil zum Injizieren mittels einer Spritze ausgebildet sein, was den Gebrauchswert der Ventilschleuse noch erhöht.

5. Die Ventilschleuse mit/ohne Temperaturfühler ist einfach aufgebaut, hat eine leichte Handhabung und besitzt eine sichere und störungsfreie Funktion.

Anhand der Zeichnungen wird nachfolgend ein Ausführungsbeispiel gemäß der Erfindung näher erläutert. Es zeigt:

Fig. 1 einen Längsschnitt durch eine Ventilschleuse mit Temperaturfühler, an die, in strichpunktierten Linien dargestellt, ein Katheter, eine Flüssigkeitszuführleitung und eine Spritze angeschlossen sind, in der Saugstellung der Spritze;

Fig. 2 einen Längsschnitt durch die Ventilschleuse nach Fig. 1, in der Druckstellung der Spritze;

Fig. 3 einen Längsschnitt durch dieselbe Ventilschleuse, jedoch ohne Temperaturfühler, in der Saugstellung der Spritze;

Fig. 4 einen Längsschnitt durch die Ventilschleuse nach Fig. 3, in der Druckstellung der Spritze.

Die erfindungsgemäße Ventilschleuse für medizinische Zwecke, insbesondere für die Herzzeitvolumenmessung und Flüssigkeitsspülung, weist ein eine Saug- und Druckkammer 2 bildendes Gehäuse 1 auf, an dem ein Anschluß 3 für einen Katheter 4, wie Venenkatheter, Einschwemmkatheter od. dgl., ein zweiter Anschluß 5 für eine Flüssigkeitszuführleitung 6, ein dritter Anschluß 7 für eine Spritze 8 und ein vierter Anschluß 9 für ein Temperatur-Meßorgan 10 vorgesehen ist.

Diese Anschlüsse 3,5,7,9 sind von an das Gehäuse 1 angeformten Rohrstutzen gebildet.

Das Gehäuse 1 ist beispielsweise im Querschnitt kreisförmig ausgebildet und hat eine zylindrische Längsform.

Die Anschlüsse 3 und 7 liegen sich an den Stirnseiten des Gehäuses 1 koaxial gegenüber und an den beiden Gehäuse-Längsseiten sind die beiden Anschlüsse 5 und 9 in Gehäuse-Längsrichtung gegeneinander versetzt gegenüberliegend angeordnet.

Dem Anschluß 5 für die Flüssigkeitszuführleitung 6 ist in der Kammer 2 ein Rückschlagventil 11 in Form einer beweglichen Klappe zugeordnet, welches an der Gehäuse-Innenwand liegt und den Kanal des Anschlusses 5 zur Kammer 2 hin absperrt oder freigibt.

Weiterhin ist dem Anschluß 3 für den Katheter 4 ein Rückschlagventil 12 in Form einer beweglichen Klappe zugeordnet, welches an einer in der Kammer 2 im Abstand zu dem Kanal des Anschlusses 3 stehenden Trennwand 13 lagert und einen Durchtritt 14 der Trennwand 13 zum Anschluß 3 hin absperrt oder freigibt; dieses Ventil 12 lagert an der dem Anschluß 3 benachbarten Trennwandseite.

In den Anschluß 9 (den Rohrstutzen) ist eine topfförmige Buchse 15 aus thermosensitivien Metall lagefixiert eingesetzt, welche in die Kammer 2 hineinragt und somit mit der in die Kammer 2 gelangenden und aus dieser wieder herauszubringenden Flüssigkeit in Berührung kommt. Diese Buchse 15 nimmt einen Temperaturfühler 10 auf, dessen elektrische Leitung 16 aus dem Anschluß 9 herausgeführt und an einem Computer angeschlossen wird. Auf den Anschluß 9 ist eine Klemmkappe 17 aufgesetzt, die den Temperaturfühler 10 und die elektrische Leitung 16 fixiert.

Der Katheter 4 und die Flüssigkeitszuführleitung 6 werden durch je eine Verbindungskappe 18 an dem Anschluß 3,5 abgedichtet festgelegt und auf dem Anschluß 7 läßt sich eine Verbindungs- oder Durchstechkappe für die Spritze 8 vorsehen, durch die die Spritze 8 gehalten und mit ihrer Nadel mindestens in den Kanal des Anschlusses 7, vorzugsweise in die Kammer 2, hineingeführt ist. Es besteht auch die Möglichkeit, an den Anschluß 7 eine Zwischenleitung (nicht dargestellt) anzuschließen und die Spritze 8 dann mit der Zwischenleitung in einem gewissen Abstand zum Gehäuse 1 zu verbinden.

Das Gehäuse 1 besteht aus Metall, Kunststoff od. dgl..

Die erfindungsgemäße Ventilschleuse arbeitet bei der Herzzeitvolumenmessung folgendermaßen:

Die Flüssigkeitszuführleitung 6 ist an einen Flüssigkeitsbehälter (nicht dargestellt) angeschlossen und der Venenkatheter 4 in die Herzkammer des Patienten verlegt.

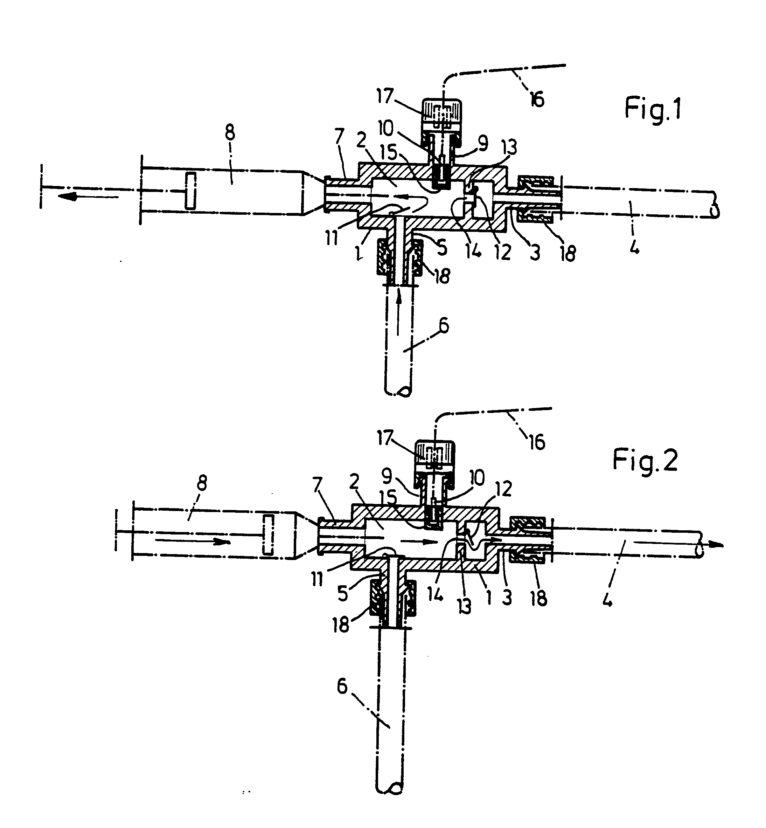

Durch Herausziehen des Kolbens der Spritze 8 erfolgt eine Aspiration und durch die in der Kammer 2 entstehende Saugwirkung wird das Ventil 11 geöffnet und gleichzeitig das Ventil 12 geschlossen und über die Leitung 6 aus dem Behälter die Flüssigkeit in die Kammer 2 gesaugt, wie in Fig. 1 dargestellt. Die in die Kammer 2 eingeströmte Flüssigkeit wird dabei direkt von der thermosensiven Metallbuchse 15 in ihrer Temperatur gemessen und über den Thermofühler 10 und die elektrische Leitung 16 wird dieser Wert sofort zum Computer zur Registrierung gegeben, so daß festgestellt wird, mit welcher Temperatur die Flüssigkeit in den Patienten injiziert wird.

Durch Einfahren des Kolbens der Spritze wird dann die Flüssigkeit aus der Kammer 2 heraus in den Katheter 4 und in die Herzkammer gedrückt, wobei das Ventil 11 geschlossen und das Ventil 12 automatisch geöffnet wird, wie in Fig. 2 dargestellt.

Der Katheter 4 steht ebenfalls mit dem Computer über einen Thermistor in Verbindung und dieser wertet nach der Eingabe des Injektionsvolumens und der Temperatur-Differenz zwischen der Injektionslösung und dem Blut des Patienten sofort die Temperatur-Zeit-Kurve aus und zeigt den ermittelten Wert des Herzzeitvolumens in Liter/Minute an.

Dieser Meßvorgang durch Aspiration und Injektion kann in gewissen Zeitabständen wiederholt werden, so daß eine genaue Bestimmung des Herzzeitvolumens möglich ist. Dabei bleibt das System geschlossen am Patienten, d.h., der Katheter 4 in die Herzkammer verlegt und der Zuführschlauch 6 sowie die Spritze 8 am Gehäuse 1 und der Temperaturfühler 10 mit seiner elektrischen Leitung 16 an den Computer angeschlossen.

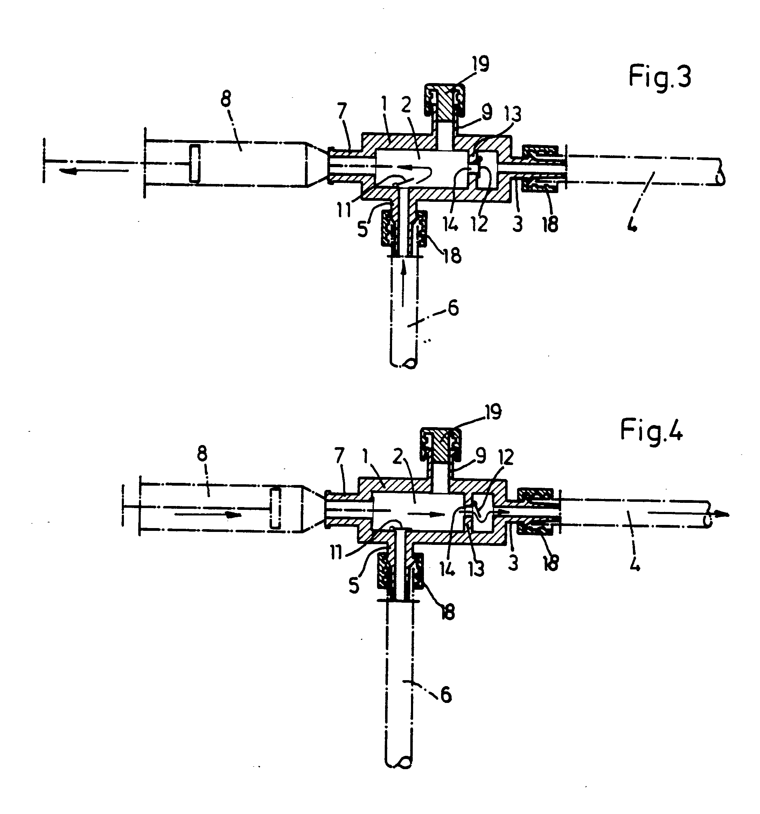

Weiterhin läßt sich die erfindungsgemäße Ventilschleuse nur für Spülvorgänge einsetzen, wobei das Gehäuse 1 ohne Buchse 15 und ohne Temperaturfühler 10 ausgestattet ist, wie in Fig. 3 und 4 dargestellt. Hierbei ist der Anschlußstutzen 9 durch einen Stopfen 19 verschlossen und bildet einen Blindanschluß. Der Anschluß kann aber auch mit einem Durchstechstopfen verschlossen sein, so daß er ein Zuspritzteil für eine Injektion bildet oder aber an den Anschluß 9 läßt sich eine Infusionsleitung anschließen. Diese Ventilschleuse bietet neben der Spülung also noch zusätzliche Injektions- oder Infusionsmöglichkeiten.

Der Spülvorgang bei der Ventilschleuse nach Fig. 3 und 4 ist derselbe wie der Aspirations- und Injektionsvorgang bei der Ventilschleuse mit Temperaturfühler 10.

## Ansprüche

1. Ventilschleuse für medizinische Zwecke, insbesondere für die Herzzeitvolumenmessung oder Flüssigkeitsspülung, gekennzeichnet **durch** ein eine Saug- und Druckkammer (2) aufweisendes Gehäuse (1) mit einem Anschluß (3) für einen Katheter (4), einem Anschluß (5) für eine Flüssigkeits-Zuführleitung (6), einem Anschluß (7) für eine Spritze (8) und einem Anschluß (9) für eine Temperatur-Meßorgan (10,15) oder eine Infusionsleitung oder ein Zuspritzteil oder eine Verschlußkappe (19), wobei dem Anschluß (5) für die Flüssigkeits-Zuführleitung (6) und dem Anschluß (3) für den Katheter (4) innerhalb der Saug- und Druckkammer (2) ein Rückschlagventil (11,12) zugeordnet ist.

2. Ventilschleuse nach Anspruch 1, dadurch gekennzeichnet, **daß** das Gehäuse (1) einen kreisförmigen Querschnitt und eine zylindrische Längsform hat, an den beiden sich gegenüberliegenden Stirnseiten die koaxial angeordneten Anschlüsse (3,7) für den Katheter (4) und die Spritze (8) und an der Längsseite die beiden sich gegenüberliegenden, jedoch in Gehäuse-Längsrichtung gegeneinander versetzt angeordneten Anschlüsse (5,9) hat.

3. Ventilschleuse nach Anspruch 1 und 2, dadurch gekennzeichnet, **daß** die Anschlüsse (3,5,7,9) von Rohrstutzen mit darauf angeordneten Kappen (17,18) für die Verbindung mit dem Katheter (4), der Zuführleitung (6), der Spritze (8) sowie dem Temperatur-Meßorgan (10) gebildet sind.

4. Ventilschleuse nach den Ansprüchen 1 bis 3, dadurch gekennzeichnet, **daß** in den Anschlußstutzen (9) eine topfförmige, in die Saug- und Meßkammer (2) hineinragende Buchse (15) aus thermosensiven Metall angeordnet ist, in der ein Temperaturfühler (10) fixiert ist, der eine mit einem Computer verbindbare und durch eine Klemmkappe (17) im Anschluß (9) festgelegte elektrische Leitung (16) aufweist.

5. Ventilschleuse nach den Ansprüchen 1 bis 4, dadurch gekennzeichnet, **daß** das Rückschlagventil (11) der Zuführleitung (6) von einer an der Kammer (2) innenseitig beweglich gehaltenen und den Kanal des Anschlußstutzens (5) freigebenden bzw. absperrenden Klappe gebildet ist.

6. Ventilschleuse nach den Ansprüchen 1 bis 5, dadurch gekennzeichnet, **daß** das Rückschlagventil (12) des Katheters (4) von einer an einer in der Kammer mit Abstand zu dem Anschluß (3) angeordneten Trennwand (13) beweglich gelagerten, und einen Durchtritt (14) in der Trennwand (13) freigebenden bzw. absperrenden Klappe gebildet ist, die an der dem Anschluß (3) benachbarten Seite der Trennwand (13) angeordnet ist.

7. Ventilschleuse nach den Ansprüchen 1 bis 6, dadurch gekennzeichnet, **daß** die beiden Rückschlagventile (11,12) in Abhängigkeit von der Aspirations- und Injektionswirkung der Spritze (8) wechselweise in die Öffnungs- und Schließstellung automatisch bewegbar sind.

8. Ventilschleuse nach den Ansprüchen 1 bis 7, dadurch gekennzeichnet, **daß** das Gehäuse (1) aus Metall und/oder Kunststoff besteht.

Fig.1

Fig.2

EP 0 293 506 A1

Fig.3

Fig.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 117 353 (DE GIRONIMO et al.) <br> * Figur 1; Seite 6, Zeilen 6-25 * | 1,7,8 | A 61 B 5/02 |
| Y | | 5,6 | |
| A | | 2 | |
| | --- | | |
| Y | US-A-3 542 026 (BLEDSOE) <br> * Figur 2 * | 5,6 | |
| | --- | | |
| Y | US-A-3 915 155 (JACOBSON et al.) <br> * Figur 1; Spalte 2, Zeilen 44-57 * | 1 | |
| | --- | | |
| Y | US-A-4 508 123 (WYATT et al.) <br> * Figur 1; Spalte 6, Zeilen 56-63 * | 1 | |
| | ----- | | |

**RECHERCHIERTE SACHGEBIETE (Int. Cl.4)**

A 61 B
A 61 M

Der vorliegende Recherchenbericht wurde für alle Patentansprüche erstellt

| Recherchenort | Abschlußdatum der Recherche | Prüfer |
|---|---|---|
| DEN HAAG | 28-01-1988 | FISCHER G.H. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
   Dokument

EPO FORM 1503 03.82 (P0403)